# EUROPÄISCHE PATENTANMELDUNG

(11) **EP 1 382 300 A1**
(43) Veröffentlichungstag der Anmeldung: **21.01.2004**
(21) Anmeldenummer: 03015301.9
(22) Anmeldetag: 07.07.2003
(51) Int. Cl.: A61B 6/08, A61B 5/055

(54) **Verfahren und Vorrichtung zur Positionierung eines Patienten in einem medizinischen Diagnose- oder Therapiegerät**

(30) Priorität: 18.07.2002 DE 10232676
(71) Anmelder: SIEMENS AKTIENGESELLSCHAFT, 80333 München (DE)
(72) Erfinder: Cherek, Dieter, 96114 Hirschaid (DE); Kagermeier, Robert, 90427 Nürnberg (DE); Loser, Michael, 91054 Erlangen (DE); Medlar, Donal, 91085 Weisendorf (DE); Steinmann, Hendrik, 67547 Worms (DE); Urmoneit, Uwe, 91466 Gerhardshofen (DE)

(57) **Zusammenfassung**

Die Erfindung betrifft ein Verfahren und eine Vorrichtung zur Positionierung eines Patienten (6) in einem medizinischen Diagnose- oder Therapiegerät, wobei ein Bildaufnahmegerät (4, 5) den Patienten (6) aufnimmt, auf einem Bildschirm (8) wiedergibt und eine Bildverarbeitung die Positionierung des Patienten (6) unterstützt. Die Erfindung zeichnet sich dadurch aus, dass durch die Bildverarbeitung automatisch mindestens eine Körperregion (14 - 17) detektiert wird und am Bildschirm (8) automatisch ein Scan-Bereich vorgeschlagen wird, der diese mindestens eine Körperregion (14 - 17) selektiv abdeckt.

## Beschreibung

Die Erfindung betrifft ein Verfahren und eine Vorrichtung zur Positionierung eines Patienten in einem medizinischen Diagnose- oder Therapiegerät, mit einer Behandlungseinheit, einer Recheneinheit und einer in mindestens einer Ebene verstellbaren Patientenliege, wobei mindestens ein Bildaufnahmegerät den Patienten aufnimmt, auf einem Bildschirm wiedergibt und eine Bildverarbeitung die Positionierung des Patienten unterstützt, wobei der Recheneinheit die räumliche Korrelation zwischen dem Koordinatensystem der Behandlungseinheit und dem Bildaufnahmegerät bekannt ist.

Ein ähnliches Verfahren und eine ähnliche Vorrichtung sind aus der Offenlegungsschrift DE 195 08 715 A1 der Anmelderin bekannt. In dieser Anmeldung wird ein medizinisches Diagnosegerät mit einem Verfahren zur Positionierung des Patienten in diesem Diagnosegerät beschrieben, wobei ein Bildaufnahmegerät eine zuvor am Patienten angebrachte Markierung erfasst und durch die Bildaufbereitung lokalisiert, so dass eine räumliche Korrelation zwischen der erfassten Position des Patienten und dem Koordinatensystem des Diagnosegerätes hergestellt werden kann, wobei anschließend die Patientenliege automatisch in eine gewünschte Untersuchungsposition verfährt. Hierbei ist es problematisch, dass zunächst auf dem Patienten eine Markierung durch den behandelnden Arzt oder entsprechend ausgebildeten medizinischen Personal angebracht werden muss. Die Anbringung solcher Markierungen auf dem Patienten können aus verschiedenen Gründen unerwünscht sein, so dass sich dieses Verfahren und die hierauf abgestimmte Vorrichtung nicht in jeder Situation eignet.

Ein weiteres, ähnliches Verfahren und eine ähnliche Vorrichtung sind aus der nachveröffentlichten deutschen Offenlegungsschrift 101 09 219 A1 bekannt. Hier wird ein bildgebendes Diagnosesystem mit einer Positioniereinrichtung beschrieben, wobei der Patienten auf der Patientenliege von mindestens einem Bildaufnahmegerät erfasst und die Abbildung auf einem Monitor dargestellt wird. Das Bedienpersonal kann nun mit Hilfe einer Bereichsauswahl-Einrichtung im Bild auf dem Bildschirm die gewünschte Untersuchungsregion manuell auswählen, indem es beispielsweise durch eine Maus oder durch einen Touch-Screen mit einem eingezeichneten Rechteck die gewünschte Untersuchungsregion kennzeichnet. Die Positioniereinrichtung fährt anschließend, aufgrund des angegebenen Scan-Bereiches, den Patienten im Diagnosesystem auf die gewünschte Position und führt in diesem Bereich die Untersuchung durch.

Eine solche Vorgehensweise erfordert vom Bedienpersonal eine sehr gute Ausbildung, da von diesem erwartet wird, dass es durch Eingabe des korrekten Scan-Bereiches die medizinischen Vorgaben korrekt umsetzen kann. Entsprechend ist dieses Verfahren auch fehleranfällig gegenüber individueller Eingabefehler und außerdem abhängig von der jeweils bedienenden Person, so dass sich bei unterschiedlichem Bedienpersonal auch unterschiedliche Scan-Bereiche zur Untersuchung der gleichen Region ergeben können.

Des weiteren ist es allgemein bekannt, dass alternativ zu den oben beschriebenen Positioniervorrichtungen und Positionierverfahren, die topogrammorientiert ausgebildet sind, auch ein Einrichten des Patienten mittels Licht- oder Laservisier möglich ist, wobei die Angabe der Länge des Scan-Bereichs durch entsprechende Lichtmarkierungen dargestellt wird, so dass das Personal direkt am Patienten die entsprechende Einstellung des Scan-Bereiches vornimmt.

In allen oben genannten Fällen ist eine manuelle, zeitaufwendige und wenig befriedigende Interaktion des Bedienpersonals erforderlich, welches insbesondere bei Reihenuntersuchungen als nachteilig anzusehen ist. Werden zusätzlich zur genaueren Positionierung auch noch Topogrammaufnahmen hergestellt, so kommt es obendrein zu einer erhöhten Strahlenbelastung des Patienten, die ebenfalls vermieden werden sollte.

Es ist daher Aufgabe der Erfindung, ein einfach durchzuführendes Verfahren zu finden, welches es auch weniger geschultem Personal ermöglicht, mit wenigen Handhabungen die gewünschte zu untersuchende oder zu behandelnde Körperregion zu erfassen und den Scan-Bereich entsprechend zu bestimmen, wobei auf die Durchführung einer zusätzlichen Topogrammaufnahme möglichst verzichtet werden sollte. Außerdem soll auch eine entsprechende Vorrichtung zur Durchführung dieses vereinfachten Verfahrens beschrieben werden.

Die oben genannte Aufgabe wird durch die Merkmale des unabhängigen Verfahrensanspruches und des unabhängigen Patentanspruches jeweils gelöst. Besondere Ausbildungen der Erfindung sind in den Unteransprüchen angegeben.

Die Erfinder haben erkannt, dass es möglich ist, durch Anwendung einer an sich bekannten Bildanalyse einer Aufnahme eines Patienten auf einer Patientenliege die einzelnen Körperregionen automatisch zu bestimmen und eine solche automatische Analyse des Bildes eines Patienten für die Positionierung in einem Diagnose- oder Therapiegerät einzusetzen. Hierbei wird das Bild eines Patienten auf einer Liege automatisch analysiert, so dass eine automatische Erfassung der einzelnen Körperregionen, wie beispielsweise Kopf, Abdomen, Brustkorb und Extremitäten aufgrund typischer Merkmale im Bild erfasst werden, so dass anschließend einzelne Scan-Regionen bestimmt werden können. Auf diese Weise ist es dem Bedienpersonal nun möglich, durch eine einfache Auswahl und/oder Benennung einer automatisch bestimmten Körperregion den Scan-Bereich zu fixieren. Hierbei sind keine tieferen anatomischen Kenntnisse des Bedienpersonals notwendig, sondern lediglich die Angabe der gewünschten zu scannenden Körperregion muss gemacht werden. Werden diese einzelnen Körperregionen bereits auf dem Bildschirm dargestellt und gegebenenfalls auch automatisch durch die Bildaufbereitung benannt, so reicht beispielsweise ein Anklicken einer entsprechenden Region oder Berührung dieser Region auf dem Touch-Screen, um anschließend den hierfür notwendigen Scan-Bereich automatisch festzulegen.

Entsprechend diesem Grundgedanken schlagen die Erfinder vor, das an sich bekannte Verfahren zur Positionierung eines Patienten in einem medizinischen Diagnose- oder Therapiegerät mit einer Behandlungseinheit, einer Recheneinheit und einer in mindestens einer Ebene verstellbaren Patientenliege, zu verbessern, wobei mindestens ein Bildaufnahmegerät den Patienten aufnimmt, auf einem Bildschirm wiedergibt und eine Bildverarbeitung die Positionierung des Patienten unterstützt, wobei der Recheneinheit die räumliche Korrelation zwischen dem Koordinatensystem der Behandlungseinheit und dem Bildaufnahmegerät bekannt ist. Die erfindungsgemäße Verbesserung dieses an sich bekannte Verfahrens besteht darin, dass durch die Bildverarbeitung automatisch mindestens eine Körperregion detektiert wird und am Bildschirm automatisch ein Scan-Bereich vorgeschlagen wird, der diese mindestens eine Körperregion selektiv abdeckt.

Bei den oben genannten Diagnose- und Therapiegeräten kann es sich beispielsweise um diagnostische Geräte, wie Röntgen-Computer-Tomographen, Kernspin-Resonanz-Tomographen, Angiographie-Röntgengeräte oder sonstige nuklear medizinische Diagnosegeräte handeln. Als Therapiegeräte kommen beispielsweise Röntgen- oder Gamma-Strahlentherapiegeräte, Lithotripter in Betracht. Entsprechend handelt es sich bei den Behandlungseinheiten jeweils um die Teilgeräte, welche die positionsrelevanten Detektoren und/oder Strahlenquellen enthalten. Als Beispiel ist die Abtasteinheit (Gantry) eines Computer-Tomographen als Behandlungseinheit zu nennen.

Zusätzlich kann die Bildverarbeitung mindestens zwei unterschiedliche Körperregionen, vorzugsweise alle auf dem Bild auffindbare Körperregionen, detektieren und je Körperregion einen Scan-Bereich automatisch vorschlagen und anzeigen.

Das Bedienpersonal muss in diesem Fall den gewünschten Scan-Bereich oder die gewünschten Scan-Bereiche lediglich anwählen, wobei es vorteilhaft sein kann, wenn die Bildverarbeitung die Bereiche der erkannten Körperregionen optisch hervorhebt. Dies kann beispielsweise durch eine farbige Markierung oder durch eine entsprechende Aufhellung oder Schraffur auf einem Schwarz-weiß-Bildschirm geschehen.

Die Auswahl eines Scan-Bereiches kann beispielsweise durch ein Bildschirmmenü oder Betätigung einer Taste vorgenommen werden.

Zusätzlich besteht auch die Möglichkeit, den von der Bildverarbeitung vorgeschlagene Scan-Bereich manuell zu korrigieren, so dass eventuelle Fehleinschätzungen der Bildverarbeitung übergangen werden können.

Des weiteren besteht auch die Option nicht nur eine einzige Körperregion sondern mehrere Körperregionen gleichzeitig anzuwählen, so dass bei einem Durchlauf des Patienten durch das Diagnose- oder Therapiegerät gleichzeitig mehrere Körperregionen gescannt werden.

Die Scan-Bereiche können beispielsweise durch zwei randständig zum Scan-Bereich angeordnete und vorzugsweise parallele Linien bestimmt werden, die dann gegebenenfalls zur Korrektur des Scan-Bereiches entsprechend am Bildschirm verschoben werden können.

Das erfindungsgemäße Verfahren kann des weiteren nicht nur in einer einzigen Ebene mit einem einzigen Bildaufnahmegerät angewendet werden, sondern es können auch zwei oder mehr Bildaufnahmegeräte verwendet werden, deren Aufnahmeachsen voneinander unabhängig, vorzugsweise orthogonal zueinander, angeordnet sein sollten. Hierdurch wird es möglich, nicht nur eine 2-dimensonale Positionierung des Patienten vorzunehmen, sondern diesen auch räumlich in drei Dimensionen zu positionieren, wobei zu jeder Bewegungsebene der Patientenliege ein Bildaufnahmegerät mit einer senkrecht zu dieser Bewegungsebene angeordneten Aufnahmeachse verwendet wird. Auf diese Weise ist es möglich, mit Hilfe von mindestens zwei Bildaufnahmegeräten die Positionierung des Patienten im 3-dimensonalen Raum weitestgehend automatisch durchzuführen, wobei sich aufgrund der automatisierten Bestimmung des Scan-Bereiches eine wesentliche Zeitersparnis ergibt, die insbesondere bei der Durchführung von Reihenuntersuchungen vorteilhaft ist.

Entsprechend dem oben beschriebenen Grundgedanken des erfindungsgemäßen Verfahrens schlagen die Erfinder auch eine Vorrichtung zur Positionierung eines Patienten in einem medizinischen Diagnose- oder Therapiegerät vor, welche eine Behandlungseinheit, eine Recheneinheit und eine in mindestens einer Ebene verstellbaren Patientenliege aufweist, wobei mindestens ein Bildaufnahmegerät für den Patienten vorgesehen ist, das auf einem Bildschirm den Patienten bildhaft wiedergibt und eine Bildverarbeitung zur Unterstützung der Positionierung des Patienten beinhaltet, wobei der Recheneinheit Informationen über die räumliche Korrelation zwischen dem Koordinatensystem der Behandlungseinheit und dem mindestens einen Bildaufnahmegerät bekannt sind. Die erfindungsgemäße Ausgestaltung dieser Vorrichtung sieht vor, dass die Bildverarbeitung Mittel zur automatischen Erkennung mindestens einer Körperregion aufweist und Mittel vorgesehen sind, die am Bildschirm automatisch einen Scan-Bereich vorschlagen, der diese mindestens eine Körperregion selektiv abdeckt.

Vorteilhaft kann die Bildverarbeitung Mittel zur Verfügung stellen, welche mindestens zwei unterschiedliche Körperregionen, vorzugsweise alle auf dem Bild des Patienten auffindbaren Körperregionen, aufweisen, wobei je Körperregion ein Scan-Bereich automatisch vorschlagen und anzeigt wird.

Entsprechend dem oben geschilderten Verfahren soll diese Vorrichtung auch Mittel, vorzugsweise vordefinierte Menüauswahlfelder oder Tasten, aufweisen, welche mindestens einen Scan-Bereich durch Auswahl einer dort definierten Körperregion bestimmen. So kann beispielsweise durch das einfache Drücken der Taste "Kopf" oder "Abdomen" der entsprechende Scan-Bereich durch die Bildverarbeitung ausgesucht werden und der entsprechende Scan-Bereich am Monitor des Systems dargestellt und der Scan entsprechend durchgeführt werden.

Trotz dieses automatischen Ablaufes der Bestimmung eines Scan-Bereiches kann zusätzlich ein Mittel zur manuellen Korrektur des von der Bildverarbeitung vorgeschlagene Scan-Bereichs integriert werden, wobei in solch einem Fall der vorgeschlagene Scan-Bereich, der meist durch einfache Linien abgegrenzt wird, durch entsprechendes Anklicken einer Maus und anschließendes Verschieben dieser Linie durch das Bedienpersonal modifiziert werden kann.

Soll die Positionierung nicht nur in einer einzigen Ebene, sondern in mehreren Ebenen, also 3-dimensonal, statt finden, so ist es notwendig, mindestens zwei Bildaufnahmegeräte vorzusehen, deren Aufnahmeachsen voneinander unabhängig, vorzugsweise orthogonal zueinander, sind. Hierdurch kann zu jeder Bewegungsebene der Patientenliege ein Bildaufnahmegerät mit einer vorzugsweise senkrecht zu dieser Bewegungsebene angeordneten Aufnahmeachse angebracht sein.

Eine weitere vorteilhafte Ausführungsvariante der erfindungsgemäßen Vorrichtung kann als Bildaufnahmegerät auch einen 3D-Scanner, vorzugsweise einen Laser-Scanner oder einen 3D-CMOS-Sensor, aufweisen, so dass das am Bildschirm dargestellte Patientenbild eine räumliche Darstellung ermöglicht. Entsprechend können auch Mittel zur räumlichen Darstellung der Scan-Bereiche vorgesehen werden.

Bei den oben genannten funktionalen Mitteln der erfindungsgemäßen Vorrichtung kann es sich im wesentlichen um Programme oder Programm-Module handeln, die in der an sich schon vorhandenen programmtechnischen Ausrüstung eines Diagnose- oder Therapiegerätes integriert werden können.

Zusätzliche Merkmale und Vorteile der Erfindung ergeben sich aus der nachfolgenden Beschreibung bevorzugter Ausführungsbeispiele unter Bezugnahme auf die Zeichnungen.

Die Erfindung soll nachfolgend anhand der Zeichnungen näher erläutert werden. Es stellen dar:
- Figur 1:: Computer-Tomograph mit Patient in Seitenansicht;
- Figur 2:: Computer-Tomograph in Draufsicht mit leerer Liege;
- Figur 3:: Computer-Tomograph in Draufsicht mit Patient;
- Figur 4:: Computer-Tomograph in Draufsicht mit automatisch ermittelten Grenzlinien;
- Figur 5:: Computer-Tomograph in Draufsicht mit erkannten Körperregionen;
- Figur 6:: Computer-Tomograph in Draufsicht mit vorgeschlagenem Scan-Bereich.

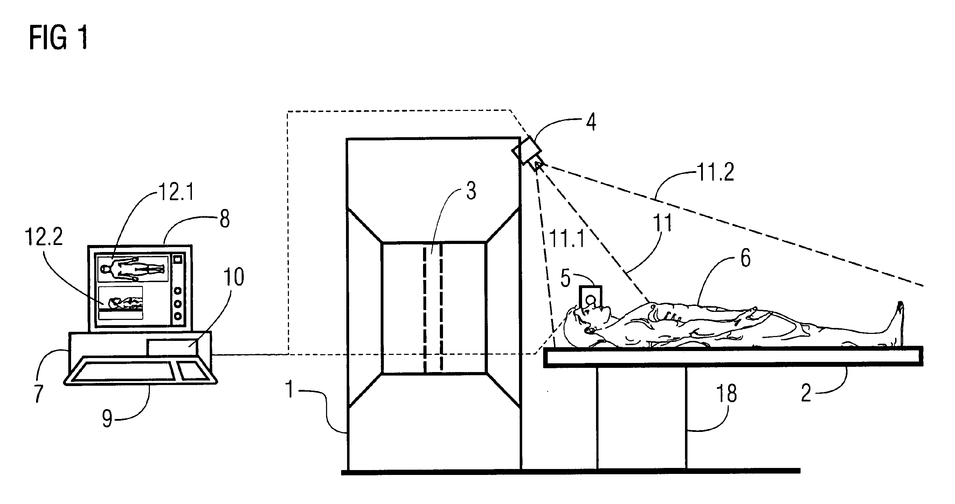

Die Figur 1 zeigt eine Seitenansicht eines Computer-Tomographen bestehend aus der Abtasteinheit (Gantry) 1, mit der in die Abtasteinheit 1 einfahrenden Liege 2 und dem Computer 7, der für die Auswertung und Steuerung des Computer-Tomographen zuständig ist.

Der Computer 7 verfügt über eine Eingabetastatur 9, einen Bildschirm 8 und einen Speicher 10, wobei über den Bildschirm 8 die Darstellung der Aufnahmen der Videokameras 4 und 5 in den Bildausschnitten 12.1 und 12.2 dargestellt werden. Die Videokamera 4 ist oberhalb des Patienten angeordnet und liefert ein Frontalbild des liegenden Patienten, während die Videokamera 5 seitlich vom Patienten angeordnet ist und dementsprechend eine seitliche Abbildung im Kopf-/Brustbereich des Patienten liefert.

Die Patientenliege 2 ist auf einer Bewegungsvorrichtung 18 angeordnet, die vom Computer 7 gezielt gesteuert werden kann, um den Patienten entsprechend in die Abtasteinheit 1 einzubringen. In diesem Zusammenhang ist anzumerken, dass aus Übersichtlichkeitsgründen lediglich die gestrichelt dargestellten Verbindungen zwischen den Videokameras 4 und 5 mit der Recheneinheit 7 dargestellt sind, wobei auf jegliche sonstige Steuer- und Datenleitungen zwischen der Abtasteinheit 1, der Bewegungsvorrichtung 18 und dem Computer 7 verzichtet wurden, um die Übersichtlichkeit der Darstellungen nicht zu verschlechtern.

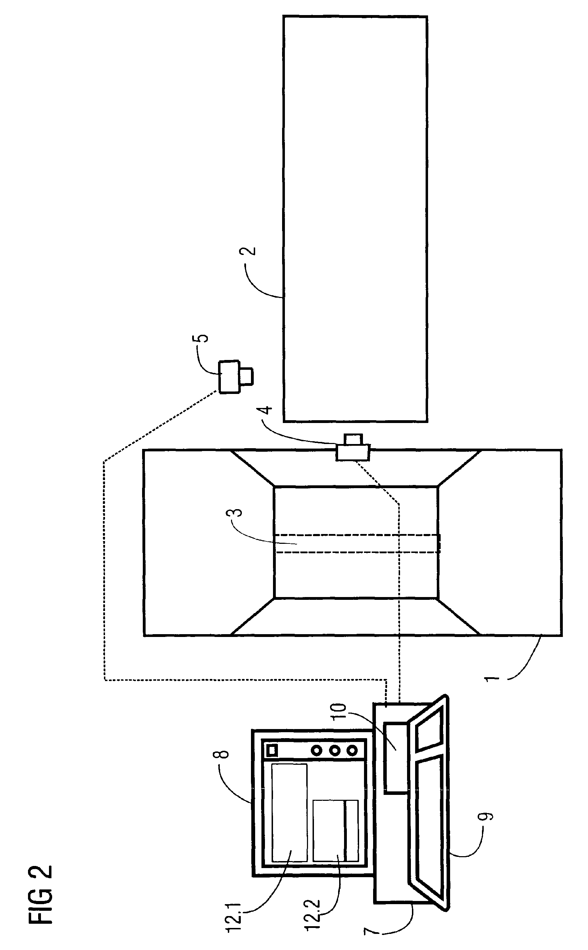

Anhand der Figuren 2 bis 5, die den Computer-Tomographen der Figur 1 in einer Draufsicht darstellen, wird nun das erfindungsgemäße Verfahren in seinen einzelnen Verfahrensschritten dargestellt.

Die Figur 2 zeigt den Computer-Tomographen mit einer leeren Patientenliege 2, der seitlich angeordneten Videokamera 5 und der oberhalb der Patientenliege angeordneten Videokamera 4. Beide Kameras können in dieser Situation eine Leeraufnahme der von ihnen erfassten Umgebung tätigen und diese in der Recheneinheit 7 mit dem dazugehörigen Speicher 10 speichern.

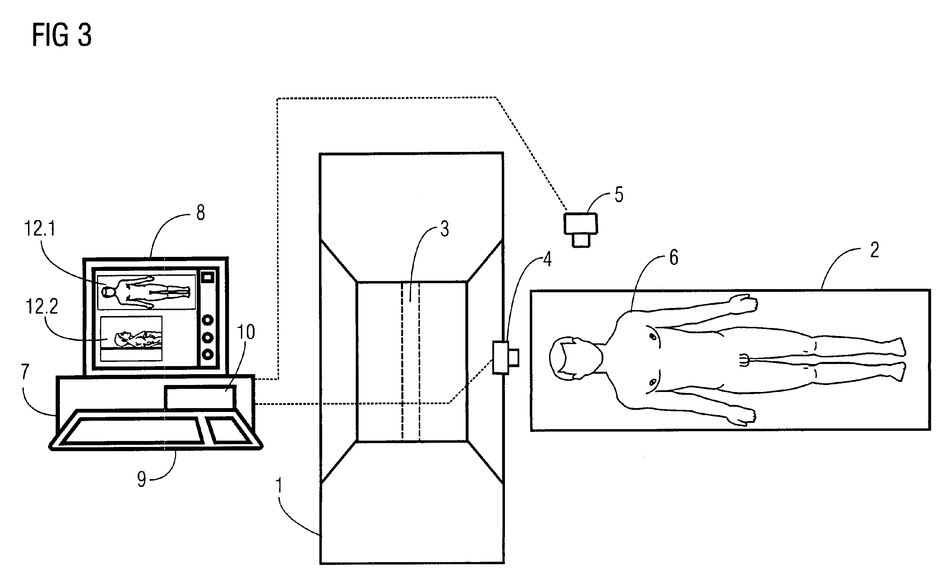

Anschließend legt sich der Patient 6, wie es in der Figur 3 gezeigt ist, auf die Patientenliege 2, hier in Rückenlage, und nimmt eine definierte Haltung ein, die beispielsweise darin bestehen kann, dass die Beine leicht gespreizt und die Hände zur Faust geballt sind, um später eine bessere Kantendetektion zu erreichen. Mit den beiden Videokameras wird nun die mit Patienten 6 besetzte Liege 2 aufgenommen und gleichzeitig die zuvor gespeicherte Leeraufnahme von der aktuellen Abbildung subtrahiert, so dass lediglich der Unterschied zwischen Leeraufnahme und der aktuellen Aufnahme als Bildinformation übrigbleibt, das heißt es ist ausschließlich der Patient in dieser Subtraktionsaufnahme zu erkennen.

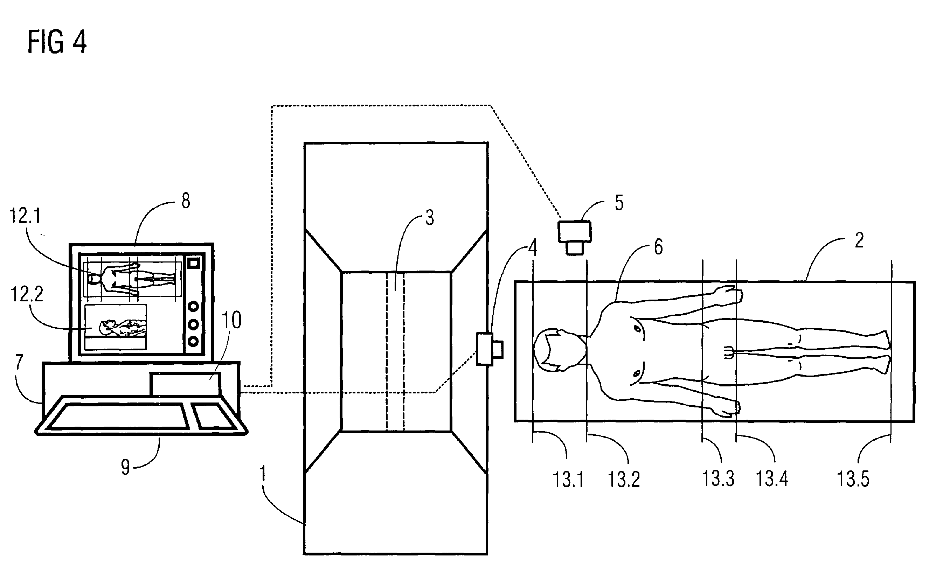

Als nächster Schritt beginnt nun die eigentliche Bildanalyse des verbliebenen Subtraktionsbildes des Patienten durch den Computer 7. Hierbei wird durch ein entsprechendes Programm die geometrische Eigenheit des Patienten analysiert, indem beispielsweise geeignete Grenzlinien 13.1 bis 13.5, wie es in der Figur 4 dargestellt ist, ermittelt werden. So ist hier beispielhaft die Grenzlinie 13.1 als oberes Ende des Kopfes und 13.2 als unteres Ende des Kopfes dargestellt. Die Linie 13.3 definiert den Bereich der Hüften, während die Grenzlinie 13.4 die Schambeingegend kennzeichnet und die Grenzlinie 13.5 das Fußende markiert.

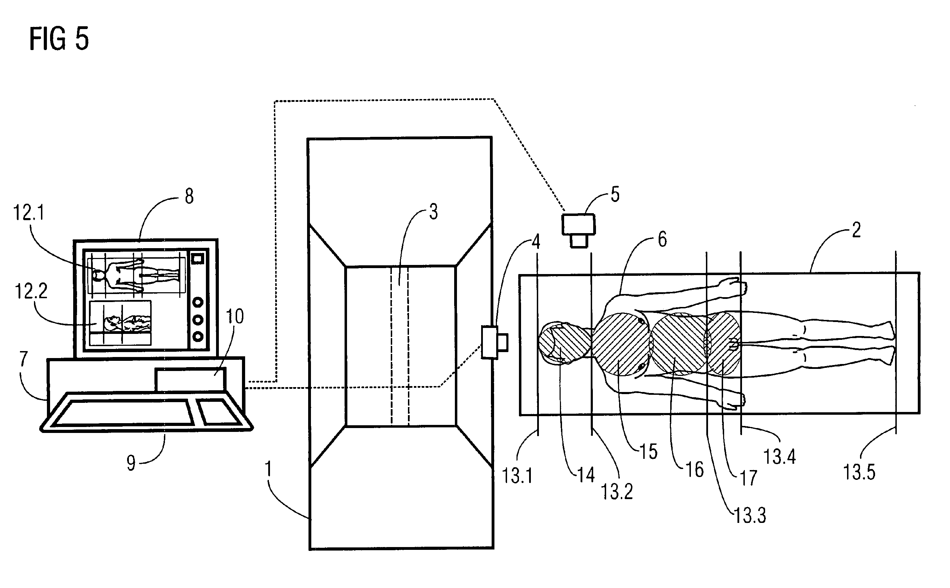

Entsprechend diesen Grenzlinien kann nun aufgrund bekannter und statistisch ermittelter Größenverhältnisse eine Einteilung in die entsprechenden Körperregionen vorgenommen werden. So zeigt die Figur 5 das Ergebnis der Analyse in den schraffiert dargestellten Bereichen 14 bis 17, entsprechend den einzelnen Körperregionen Kopf, Thorax, Abdomen und Unterbauch. Zur besseren Ansicht wurden diese Körperregionen 14 bis 17 zusätzlich direkt auf dem Patienten dargestellt, wobei jedoch in der realen Ausführung diese Regionen nur auf dem Bildschirm 8 dargestellt werden. Gleiches gilt für die eingezeichneten Grenzlinien 13.1 bis 13.5.

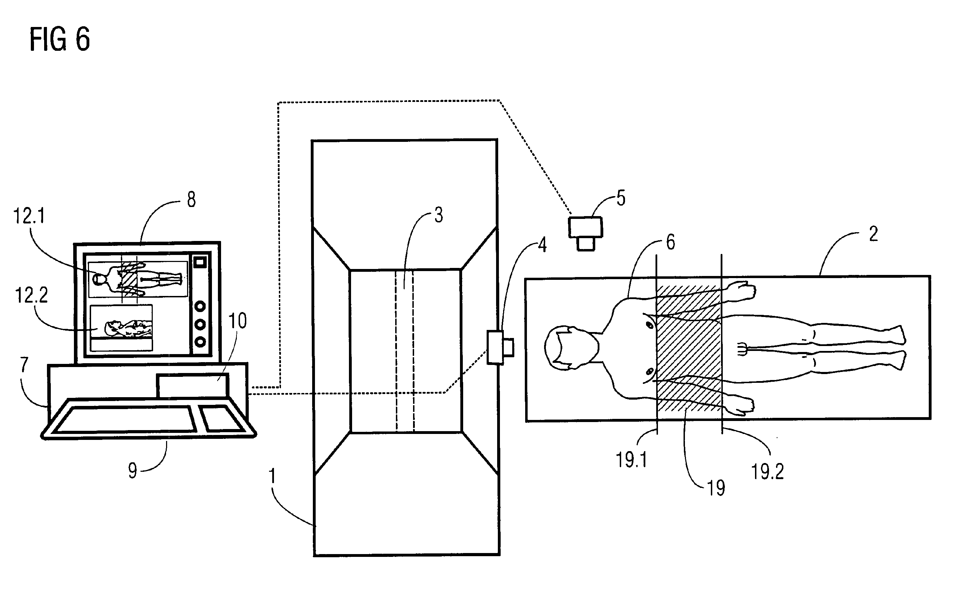

Das Bedienpersonal hat nun die Möglichkeit, durch einfache Betätigung einer Taste oder durch Anklicken in einem Bildschirmmenü eine oder mehrere Körperregionen auszuwählen, worauf anschließend diese Körperregion oder Körperregionen als Scan-Bereich 19 mit den Begrenzungen 19.1 und 19.2, wie es in Figur 6 dargestellt ist, durch den Computer 7 markiert werden. Da der Computer 7 die räumliche Korrelation zwischen den Videokameras kennt, kann er nun den Patienten automatisch in die Abtasteinrichtung einschieben, so dass die vorgewählte(n) Körperregion(en), hier das Abdomen, entsprechend abgescannt werden.

Eine Variante des geschilderten Verfahrens kann auch darin bestehen, dass vor der Durchführung der Bildanalyse an der Eingabevorrichtung 9 die gewünschte abzubildende Körperregion benannt wird oder durch ein entsprechendes Menü oder einer Taste bestimmt wird, so dass der Computer anschließend lediglich die vorbestimmte Körperregion in der Aufnahme des Patienten sucht und ausschließlich diese als mögliche Scan-Region angezeigt wird.

Des weiteren besteht auch die Möglichkeit nach der automatischen Definition des zu scannenden Bereiches die Grenzen 19.1 und 19.2 des Scan-Bereiches manuell durch entsprechende Eingabevorrichtungen, beispielsweise eine Maus oder ein Joy-Stick oder einen Touch-Screen, zu verschieben und damit zu korrigieren.

Es ist noch darauf hinzuweisen, dass die seitlich angebrachte Videokamera 5 im wesentlichen dazu dient, die richtige Höhenlage des Patienten in Relation zum Rotationszentrum des Detektors einzustellen. Dieses kann rein manuell oder durch eine automatische Bildanalyse geschehen, jedoch kann auch zusätzlich die dort gewonnene Information über die Kontur des Patienten dazu verwendet werden, einzelne Körperregionen mit besserer Genauigkeit zu bestimmen.

Des weiteren wird auch noch darauf hingewiesen, dass anstelle einer oder mehrerer Videokameras auch eine dreidimensionale Konturabtastung, beispielsweise mit Hilfe eines Laser-Scaners, vorzugsweise mit einem 3D-CMOS-Sensor, vorgenommen werden kann, so dass eine entsprechende räumliche Darstellung auf dem Bildschirm 8 angezeigt werden kann, wobei durch eine räumliche Abbildung des Patienten die Erkennung einzelner Körperregionen zusätzlich verbessert wird.

Ausdrücklich wird darauf hingewiesen, dass das erfindungsgemäße Verfahren sich nicht nur auf Röntgen-Computer-Tomographen, sondern auch auf Kernspin-Tomographen, Angiographie-Einrichtungen, Ultraschalltherapiegeräte oder Strahlentherapiegeräte anwenden lässt, ohne den Rahmen der Erfindung zu verlassen. Entsprechend sind auch solche Positioniervorrichtungen an diesen genannten Geräten mit eingeschlossen.

Es versteht sich, dass die vorstehend genannten Merkmale der Erfindung nicht nur in der jeweils angegebenen Kombination, sondern auch in anderen Kombinationen oder in Alleinstellung verwendbar sind, ohne den Rahmen der Erfindung zu verlassen.

## Patentansprüche

1. Verfahren zur Positionierung eines Patienten (6) in einem medizinischen Diagnose- oder Therapiegerät mit einer Behandlungseinheit (1), einer Recheneinheit (7) und einer in mindestens einer Ebene verstellbaren Patientenliege (2), wobei mindestens ein Bildaufnahmegerät (4, 5) den Patienten (6) aufnimmt, auf einem Bildschirm (8) wiedergibt und eine Bildverarbeitung die Positionierung des Patienten (6) unterstützt, wobei der Recheneinheit (7) die räumliche Korrelation zwischen dem Koordinatensystem der Behandlungseinheit (1) und dem Bildaufnahmegerät bekannt ist,
**dadurch gekennzeichnet, dass** durch die Bildverarbeitung automatisch mindestens eine Körperregion (14 - 17) detektiert wird und am Bildschirm (8) automatisch ein Scan-Bereich vorgeschlagen wird, der diese mindestens eine Körperregion (14 - 17) selektiv abdeckt.

2. Verfahren gemäß dem voranstehenden Patentanspruch 1, **dadurch gekennzeichnet, dass** die Bildverarbeitung mindestens zwei unterschiedliche Körperregionen (14 - 17), vorzugsweise alle auffindbare Körperregionen (14 - 17), detektiert und je Körperregion (14 - 17) einen Scan-Bereich (19) automatisch vorschlägt und anzeigt.

3. Verfahren gemäß einem der voranstehenden Patentansprüche 1 bis 2,
**dadurch gekennzeichnet, dass** die Bildverarbeitung die Bereiche der erkannten Körperregionen (14 - 17) optisch hervorhebt.

4. Verfahren gemäß einem der voranstehenden Patentansprüche 1 bis 3,
**dadurch gekennzeichnet, dass** der mindestens eine Scan-Bereich (19) durch Auswahl einer Körperregion (14 - 17) auf einem Bildschirmmenü oder Betätigung einer Taste bestimmt wird.

5. Verfahren gemäß einem der voranstehenden Patentansprüche 1 bis 4,
**dadurch gekennzeichnet, dass** der von der Bildverarbeitung vorgeschlagene Scan-Bereich (19) manuell korrigiert werden kann.

6. Verfahren gemäß einem der voranstehenden Patentansprüche 1 bis 5,
**dadurch gekennzeichnet, dass** mehr als eine Körperregion (14 - 17) gleichzeitig anwählbar ist.

7. Verfahren gemäß einem der voranstehenden Patentansprüche 1 bis 6,
**dadurch gekennzeichnet, dass** der mindestens eine Scan-Bereich (19) durch zwei randständig zum Scan-Bereich (19.1, 19.2) angeordnete und vorzugsweise parallele Linien bestimmt wird.

8. Verfahren gemäß einem der voranstehenden Patentansprüche 1 bis 7,
**dadurch gekennzeichnet, dass** mindestens zwei Bildaufnahmegeräte (4, 5) verwendet werden, deren Aufnahmeachsen (11) voneinander unabhängig, vorzugsweise orthogonal zueinander, sind.

9. Verfahren gemäß dem voranstehenden Patentanspruch 8, **dadurch gekennzeichnet, dass** zu jeder Bewegungsebene der Patientenliege (2) ein Bildaufnahmegerät mit einer senkrecht zu dieser Bewegungsebene angeordneten Aufnahmeachse (11) verwendet wird.

10. Vorrichtung zur Positionierung eines Patienten in einem medizinischen Diagnose- oder Therapiegerät, vorzugsweise zur Durchführung des Verfahrens eines der vorgenannten Verfahrensansprüche, mit einer Behandlungseinheit (1), einer Recheneinheit (7) und einer in mindestens einer Ebene verstellbaren Patientenliege (2), wobei mindestens ein Bildaufnahmegerät (4, 5) für den Patienten (6) vorgesehen ist, das auf einem Bildschirm (8) den Patienten (6) bildhaft wiedergibt und eine Bildverarbeitung zur Unterstützung der Positionierung des Patienten (6) vorgesehen ist, wobei die Recheneinheit (7) Informationen über die räumliche Korrelation zwischen dem Koordinatensystem der Behandlungseinheit (1) und dem mindestens einen Bildaufnahmegerät (4, 5) bekannt ist, **dadurch gekennzeichnet, dass** die Bildverarbeitung Mittel zur automatischen Erkennung mindestens einer Körperregion (14 - 17) aufweist und Mittel vorgesehen sind, die am Bildschirm (8) automatisch einen Scan-Bereich (13.1-13.2, 13.2-13.3, 13.3-13.4, 13.4-13.5) vorgeschlagen, der diese mindestens eine Körperregion (14 - 17)selektiv abdeckt.

11. Vorrichtung gemäß dem voranstehenden Patentanspruch 10,
**dadurch gekennzeichnet, dass** in der Bildverarbeitung Mittel zur Erkennung mindestens zwei unterschiedlicher Körperregionen (14 - 17), vorzugsweise aller auf dem Bild des Patienten (12.1, 12.2) auffindbaren Körperregionen (14 - 17), vorgesehen sind, welche je Körperregion (14 - 17) einen Scan-Bereich (19) automatisch vorschlagen und anzeigen.

12. Vorrichtung gemäß einem der voranstehenden Patentansprüche 10 bis 11,
**dadurch gekennzeichnet, dass** Mittel, vorzugsweise vordefinierte Menüauswahlfelder oder Tasten, vorgesehen sind, welche mindestens einen Scan-Bereich (19) durch Auswahl einer dort definierten Körperregion (14 - 17) bestimmen.

13. Vorrichtung gemäß einem der voranstehenden Patentansprüche 10 bis 12,
**dadurch gekennzeichnet, dass** Mittel zur manuellen Korrektur des von der Bildverarbeitung vorgeschlagene Scan-Bereichs (19) vorgesehen sind.

14. Vorrichtung gemäß einem der voranstehenden Patentansprüche 10 bis 13,
**dadurch gekennzeichnet, dass** mindestens zwei Bildaufnahmegeräte (4, 5) vorgesehen sind, deren Aufnahmeachsen (11) voneinander unabhängig, vorzugsweise orthogonal zueinander, sind.

15. Vorrichtung gemäß dem voranstehenden Patentanspruch 14,
**dadurch gekennzeichnet, dass** zu jeder Bewegungsebene der Patientenliege (2) ein Bildaufnahmegerät (4, 5) mit einer senkrecht zu dieser Bewegungsebene angeordneten Aufnahmeachse (11) angebracht ist.

16. Vorrichtung gemäß einem der voranstehenden Patentansprüche 10 bis 15,
**dadurch gekennzeichnet, dass** mindestens ein Bildaufnahmegerät ein 3D-Scanner, vorzugsweise mit einem Laser-Scanner oder einem 3D-CMOS-Sensor, ist.

17. Vorrichtung gemäß dem voranstehenden Patentanspruch 16,
**dadurch gekennzeichnet, dass** das am Bildschirm dargestellte Patientenbild (12.1, 12.2) eine räumliche Darstellung, gegebenenfalls mit entsprechender 3D-Brille beim Bedienungspersonal, ermöglicht.

18. Vorrichtung gemäß dem voranstehenden Patentanspruch 17,
**dadurch gekennzeichnet, dass** Mittel zur räumlichen Darstellung der Scan-Bereiche vorgesehen sind.

19. Vorrichtung gemäß einem der voranstehenden Patentansprüche 10 bis 18,
**dadurch gekennzeichnet, dass** die genannten Mittel Programme oder Programm-Module darstellen.
